# EUROPÄISCHE PATENTANMELDUNG

(11) **EP 3 069 699 A1**
(43) Veröffentlichungstag der Anmeldung: **21.09.2016**
(21) Anmeldenummer: 16159202.7
(22) Anmeldetag: 08.03.2016
(51) Int. Cl.: A61F 7/02

(54) **WÄRMEBEHANDLUNGSVORRICHTUNG ZUR VERWENDUNG BEI DER WÄRMETHERAPEUTISCHEN BEHANDLUNG DES MENSCHLICHEN KÖRPERS**

(30) Priorität: 17.03.2015 DE 102015003437
(71) Anmelder: Hilotherm Holding AG, 6317 Oberwil bei Zug (CH)
(72) Erfinder: Janisch, Klaus Johann, 88316 Isny (DE)
(74) Vertreter: Wallinger, Michael

(57) **Zusammenfassung**

Die Erfindung betrifft eine Wärmebehandlungsvorrichtung zur Verwendung bei der wärmetherapeutischen Behandlung eines Teiles des menschlichen Körpers mit einem Wärmeübertragungskörper 1 mit zwei flexiblen Materiallagen 11, 12, welche zwischen sich einen oder mehrere von einem wärmeübertragenden Fluid durchströmbare Strömungsräume 4 begrenzen, sowie mit wenigstens einer ersten Zuleitung 2 zur Zuführung und wenigstens einer zweiten Zuleitung 3 zur Abführung eines wärmeübertragenden Fluids zu bzw. von dem Wärmeübertragungskörper 1.

Der Wärmeübertragungskörper 1 weist wenigstens eine erste Form auf oder kann eine solche erste Form annehmen, in welcher er den zu behandelnden Körperteil zumindest teilweise umschließt. Der zu behandelnde Körperteil ist vorzugsweise eine Hand und die erste Form eine Handschuhform.

## Beschreibung

Die vorliegende Erfindung betrifft eine Wärmebehandlungsvorrichtung und ein System zur Verwendung bei thermotherapeutischen Behandlungen.

Die thermotherapeutische Behandlung ist ein schon sehr lange bekanntes medizinisches Heilverfahren, bei dem dem Körper Wärme zugeführt wird - man spricht dann von Wärmetherapie - oder dem Körper Wärme entzogen wird - in diesem Fall spricht man von Kältetherapie. Um bei der Wärmetherapie Wärme auf den Körper übertragen zu können, ist die Wärmeübertragung von einer Wärmequelle auf den Körper erforderlich. Bei der Kältetherapie muss hingegen die Wärme dem Körper entzogen werden, d. h. es muss Wärme des Körpers auf eine Wärmesenke übertragen werden.

Die Wärmeübertragung kann in beiden Fällen direkt erfolgen, d. h. indem der Körper selbst in Kontakt mit einem wärmeübertragenden Medium gebracht wird, z. B. in einer Sauna oder in einem kalten Wasserbad. Daneben ist eine indirekte Wärmeübertragung möglich, bei welcher der Körper nicht selbst in Kontakt mit einem wärmeübertragenden Medium kommt. Die vorliegende Erfindung befasst sich mit der letztgenannten Vorgehensweise.

Bei der indirekten Wärmeübertragung wird ein Wärmeübertragungsmedium verwendet, das je nach Anwendung gasförmig oder flüssig, aber auch fest sein kann. Besonders bevorzugt ist als Wärmeübertragungsmedium Wasser, insbesondere destilliertes Wasser. Daneben können aber auch ein Gas oder eine Gasmischung und hier insbesondere Luft oder auch ein Öl oder ein Ölgemisch oder andere geeignete Flüssigkeiten oder Flüssigkeitsgemische zur Anwendung kommen.

Der Prozess der Wärmeübertragung kann diskontinuierlich oder kontinuierlich erfolgen. Eine bekannte diskontinuierliche Wärmeübertragungsanwendung ist der sogenannte Eisbeutel. Dazu wird Wasser zu Eis gefroren und in eine geeignete flexible, meist wasserdicht verschließbare Hülle eingefüllt. Der Eisbeutel wird auf den jeweiligen Körperteil aufgelegt und entzieht dem Körper so lange Wärme, bis das Eis aufgetaut ist und sich die Temperatur des Eisbeutels der Körpertemperatur angenähert hat.

Beim kontinuierlichen Verfahren wird das wärmeübertragende Medium mit einer Temperatur, die je nach Anwendung oberhalb oder unterhalb der Körpertemperatur liegt, einem Wärmeübertragungskörper zugeführt, der in Kontakt mit dem betroffenen Körperteil steht.

Der vorliegenden Erfindung liegt die Aufgabe zugrunde, eine Wärmebehandlungsvorrichtung mit wenigstens einem Wärmeübertragungskörper und ein System mit einer solchen Wärmebehandlungsvorrichtung zu schaffen, wobei der Wärmeübertragungskörper, je nach Gestaltung, für die kontinuierliche, die diskontinuierliche oder aber für beide Therapieformen geeignet ist.

Diese Aufgabe wird erfindungsgemäß durch den Gegenstand des Anspruchs 1 gelöst. Zu bevorzugende Weiterbildungen der Erfindung sind Gegenstand der Unteransprüche.

Das erfindungsgemäße System ist Gegenstand des Anspruches 18.

Die Erfindung sieht eine Wärmebehandlungsvorrichtung zur Verwendung bei der wärmetherapeutischen Behandlung eines Teiles des menschlichen Körpers vor, insbesondere zur Verwendung bei der Behandlung von Gewebeschädigungen nach operativen Eingriffen, von Verletzungen, von Entzündungen und von chronischen Erkrankungen des rheumatischen Formenkreises, mit einem Wärmeübertragungskörper, welcher aufweist:
- wenigstens eine erste flexible Materiallage mit wenigstens einer ersten Hautkontaktfläche, welche zumindest teilweise dem zu behandelnden Körperteil zugewandt ist, und mit wenigstens einer von der ersten Hautkontaktfläche abgewandten ersten Fluidkontaktfläche,
- wenigstens eine zweite flexible Materiallage mit wenigstens einer zweiten Fluidkontaktfläche, die zwischen sich und der ersten Fluidkontaktfläche der ersten flexiblen Materiallage zumindest teilweise einen oder mehrere von einem wärmeübertragenden Fluid durchströmbare Strömungsräume begrenzt,
wobei die Wärmebehandlungsvorrichtung weiterhin aufweist:
- wenigstens eine erste Zuleitung, um dem Wärmeübertragungskörper ein wärmeübertragendes Fluid zuzuführen,
- wenigstens eine zweite Zuleitung, um ein wärmeübertragendes Fluid von dem Wärmeübertragungskörper abzuführen.

Dabei weist der Wärmeübertragungskörper wenigstens eine erste Form auf oder kann eine solche erste Form annehmen, in welcher er den zu behandelnden Körperteil zumindest teilweise umschließt.

Auf diese Weise ist sichergestellt, dass die erste Hautkontaktfläche an dem zu behandelnden Körperteil flächig anliegt und damit eine gute Wärmeübertragung gewährleistet ist.

Unter einem Strömungsraum wird hierbei ein durchgehender Raum verstanden, in dem das wärmeübertragende Fluid für je zwei beliebige Punkte im Inneren des Raumes von einem der Punkte zu dem anderen Punkt strömen kann.

Die Wärmebehandlungsvorrichtung kann zum einmaligen oder auch zum mehrmaligen Gebrauch vorgesehen sein.

In einer bevorzugten Ausführung der Erfindung ist der zu behandelnde Körperteil eine Hand, und der Wärmeübertragungskörper weist eine Handschuhform auf oder kann eine Handschuhform annehmen.

In einer bevorzugten Variante dieser Ausführung weist der Wärmeübertragungskörper eine der folgenden Formen auf oder kann eine dieser Formen annehmen:
- die Form einer die Hand umschließenden Hülle ohne Unterteilungen des Innenraums;
- die Form eines Handschuhs mit teilweise abgeteilten Umhüllungen für wenigstens einen Finger, insbesondere die Form eines Fausthandschuhs mit einer teilweise abgeteilten Umhüllung für den Daumen;
- die Form eines Fingerhandschuhs mit teilweise abgeteilten Umhüllungen für jeden Finger;
- die Form eines zumindest teilweise fingerlosen Handschuhs, bei dem wenigstens ein Finger nicht umschlossen ist.

Unter einer Handschuhform im Sinne der vorliegenden Erfindung sollen auch weitere Formen verstanden werden, bei denen wenigstens ein Teil der Hand, insbesondere der Handrücken, die Handinnenfläche, der Handballen, das Handgelenk, ein Finger oder ein Teil eines Fingers, umschlossen ist.

Der zu behandelnde Körperteil kann jedoch auch ein Fuß oder ein anderer Körperteil sein, wobei der Wärmeübertragungskörper dann eine Schuh- oder Strumpfform bzw. eine andere an den jeweiligen Körperteil angepasste Form aufweist oder eine solche Form annehmen kann.

In einer weiteren bevorzugten Ausführung der Erfindung ist innerhalb wenigstens eines Strömungsraumes wenigstens ein auf Zug und Druck beanspruchbares Verbindungselement zwischen der ersten und der zweiten Fluidkontaktfläche angeordnet.

Derartige Verbindungselemente, welche insbesondere in kleinen und regelmäßigen Abständen auf der ersten und der zweiten Fluidkontaktfläche verteilt sind, dienen insbesondere dazu, ein Verschließen der Strömungsräume durch ein flächiges Aufeinanderliegen der ersten und der zweiten Fluidkontaktfläche zu verhindern. Die Verbindungselemente sind vorzugsweise als Schweißpunkte ausgeführt, mit denen die erste und die zweite flexible Materiallage miteinander verschweißt und dadurch verbunden sind.

In einer bevorzugten Variante dieser Ausführung weist der Wärmeübertragungskörper eine Vielzahl von Verbindungselementen auf, welche zumindest teilweise in einem, vorzugsweise rechteckigen, weiter vorzugsweise quadratischen, Raster zwischen der ersten und der zweiten Fluidkontaktfläche angeordnet sind.

Ein solches Raster erleichtert das Design des Wärmeübertragungskörpers und sorgt dafür, dass ein Verschließen der Strömungsräume auf der ersten und zweiten Fluidkontaktfläche oder auf Teilen davon gleichmäßig verhindert wird.

In einer weiteren bevorzugten Ausführung der Erfindung sind zwischen der ersten und der zweiten Fluidkontaktfläche verlaufende Begrenzungen der Strömungsräume zumindest teilweise durch linienförmige Kontaktierungen der ersten Fluidkontaktfläche mit der zweiten Fluidkontaktfläche gebildet.

Derartige linienförmige Kontaktierungen lassen sich vorzugsweise auf besonders einfache und sichere Weise durch Verschweißen der ersten flexiblen Materiallage mit der zweiten flexiblen Materiallage als Schweißnähte herstellen und erfordern dann keine weiteren, separaten Elemente mehr zur Begrenzung der Strömungsräume.

In einer bevorzugten gemeinsamen Variante dieser Ausführung der Erfindung und der oben beschriebenen Ausführung, welche Verbindungselemente zwischen der ersten und der zweiten Fluidkontaktfläche vorsieht, sind zumindest zwei benachbarte Verbindungselemente, insbesondere die Mittelpunkte der beiden Verbindungselemente, durch eine der linienförmigen Kontaktierungen, insbesondere geradlinig, verbunden.

Eine solche sich "überlagernde" Anordnung der linienförmigen Kontaktierungen zur Begrenzung der Strömungsräume und der Verbindungselemente ist vorteilhaft, da sonst Verbindungselemente sehr nah an den linienförmigen Kontaktierungen zu liegen kommen könnten und die durchströmbare Breite des Strömungsraumes an dieser Stelle unnötig stark eingeschränkt würde. Außerdem verhindern auch die linienförmigen Kontaktierungen selbst einen Verschluss der Strömungsräume, so dass eine unmittelbar benachbarte Anordnung von linienförmigen Kontaktierungen und Verbindungselementen unnötig ist.

Weiterhin bewirkt die "überlagernde" Anordnung von linienförmigen Kontaktierungen und Verbindungselementen - insbesondere dann, wenn letztere in einem Raster angeordnet sind - einen übersichtlichen und optisch ansprechenden Verlauf der Strömungsräume.

In einer bevorzugten Variante dieser Ausführung weist wenigstens eine der linienförmigen Kontaktierungen wenigstens einen geradlinigen Abschnitt auf. Vorzugsweise bestehen die linienförmigen Kontaktierungen zu mehr als 50 % ihrer Gesamtlänge, weiter vorzugsweise zu mehr als 90 % ihrer Gesamtlänge aus geradlinigen Abschnitten.

Dadurch sind die Werkzeuge zur Herstellung des Wärmeübertragungskörpers, insbesondere Werkzeugteile zum Pressen und Verschweißen der ersten und zweiten flexiblen Materiallage miteinander entlang der linienförmigen Kontaktierungen, einfacher herzustellen. Auch in diesem Fall ergibt sich wieder ein übersichtlicher und optisch ansprechender Verlauf der Strömungsräume.

In einer weiteren bevorzugten Ausführung der Erfindung ist der Wärmeübertragungskörper zusammenklappbar, insbesondere weist er zwei im Wesentlichen zueinander symmetrisch geformte Hälften auf, welche im zusammengeklappten Zustand des Wärmeübertragungskörpers im Wesentlichen miteinander in Deckung gebracht sind.

Vorzugsweise nimmt der Wärmeübertragungskörper im zusammengeklappten Zustand die erste Form an, in der er den zu behandelnden Körperteil zumindest teilweise umschließt.

Durch die Zusammenklappbarkeit ist der Wärmeübertragungskörper leichter herstellbar, insbesondere in Form einer ebenen Fläche, in welcher die erste und die zweite flexible Materiallage miteinander verbunden werden. Weiterhin ist insbesondere die erste Hautkontaktfläche im aufgeklappten Zustand des Wärmeübertragungskörpers leichter zu reinigen und/oder zu desinfizieren als im zusammengeklappten Zustand.

In einer bevorzugten Variante dieser Ausführung weist der Wärmeübertragungskörper einen faltenartigen Übergangsbereich mit einer Faltenlinie auf, wobei der Wärmeübertragungskörper entlang der Faltenlinie zusammenklappbar ist und sich wenigstens ein Strömungsraum über die Faltenlinie hinweg erstreckt.

Unter einem faltenartigen Übergangsbereich wird hierbei ein Bereich des Wärmeübertragungskörpers verstanden, in dem die erste und die zweite flexible Materiallage im zusammengeklappten Zustand des Wärmeübertragungskörpers im Querschnitt eine Biegung mit einem kleinen Radius aufweisen. Die Faltenlinie ist dann die Verbindungslinie der Scheitelpunkte dieser Biegung. Falls das Material der ersten und der zweiten flexiblen Materiallage nicht oder nur wenig elastisch ist, ist die Faltenlinie im Allgemeinen eine gerade Linie.

Dass sich ein Strömungsraum über die Faltenlinie hinweg erstreckt, hat den Vorteil, dass sich der gesamte Wärmeübertragungskörper dann mit nur einem durchgehenden Strömungsraum realisieren lässt und somit auch nur eine erste Zuleitung und eine zweite Zuleitung, welche auch keine Verzweigungen zu verschiedenen Strömungsräumen aufweisen müssen, nötig sind.

Bevorzugt erstrecken sich mehrere Strömungsräume oder mehrere Abschnitte desselben Strömungsraumes über die Faltenlinie hinweg.

Bevorzugt sind mehrere sich über die Faltenlinie hinweg erstreckende Abschnitte desselben Strömungsraumes auf einer oder auf beiden Seiten der Faltenlinie durch je einen Abschnitt dieses Strömungsraumes verbunden, welcher jeweils im Wesentlichen entlang der Faltenlinie verläuft.

Bevorzugt verläuft ein sich über die Faltenlinie hinweg erstreckender Strömungsraum gleichzeitig auf beiden Seiten der Faltenlinie im Wesentlichen entlang der Faltenlinie.

Bevorzugt ist wenigstens ein Verbindungselement auf der Faltenlinie innerhalb eines Strömungsraumes, welcher sich über die Faltenlinie hinweg erstreckt, angeordnet.

Bevorzugt ist die entlang der Faltenlinie gemessene Gesamtbreite eines sich über die Faltenlinie hinweg erstreckenden Strömungsraumes größer als, insbesondere wenigstens doppelt so groß wie, die senkrecht zur Strömungsrichtung gemessene Gesamtbreite dieses Strömungsraumes in einem in Strömungsrichtung gesehen unmittelbar vor oder nach dem faltenartigen Übergangsbereich angeordneten Abschnitt dieses Strömungsraumes.

Die genannten Maßnahmen dienen - einzeln oder in Kombination - dazu, die Querschnittsfläche des oder der sich über die Faltenlinie hinweg erstreckenden Strömungsräume gezielt zu vergrößern, um einer Verringerung der Höhe der Strömungsräume, welche durch das Zusammenklappen des Wärmeübertragungskörpers entlang der Faltenlinie verursacht werden kann, entgegenzuwirken. Durch eine solche Höhenverringerung der Strömungsräume könnte die Menge des wärmeübertragenden Fluids, welche pro Zeiteinheit durch die Strömungsräume strömen kann, und damit auch die Wärmeübertragung selbst verringert werden.

In einer weiteren bevorzugten Ausführung der Erfindung sind wenigstens zwei Abschnitte, insbesondere zwei im Wesentlichen zueinander symmetrisch geformte Hälften, des Wärmeübertragungskörpers durch wenigstens ein Fixierungselement, insbesondere durch einen Druckknopf, einen Klettverschluss oder eine Schlaufe zum Binden, miteinander lösbar verbindbar.

Dadurch kann der Wärmeübertragungskörper wiederholt geöffnet, gereinigt und/oder desinfiziert sowie wieder verschlossen werden, wodurch der Zeitraum der Verwendbarkeit der Wärmebehandlungsvorrichtung verlängert werden kann.

Es ist jedoch auch möglich, dass wenigstens zwei Abschnitte des Wärmeübertragungskörpers unlösbar, beispielsweise durch Verschweißen, miteinander verbunden sind. Dies erhöht die Stabilität der Wärmebehandlungsvorrichtung und die Wärmeisolation des zu behandelnden Körperteils gegenüber der Umgebung.

Vorzugsweise erfolgt die Verbindung der wenigstens zwei Abschnitte des Wärmeübertragungskörpers miteinander im zusammengeklappten Zustand des Wärmeübertragungskörpers. Vorzugsweise nimmt der Wärmeübertragungskörper durch das Verbinden der wenigstens zwei Abschnitte die erste Form an, in welcher er den zu behandelnden Körperteil zumindest teilweise umschließt, insbesondere eine Handschuhform.

In einer bevorzugten Variante dieser Ausführung sind die wenigstens zwei Abschnitte des Wärmeübertragungskörpers auf verschiedene Weise miteinander lösbar verbindbar, so dass dabei verschiedene Größen des Wärmeübertragungskörpers entstehen, welche insbesondere verschiedenen Größen des zu behandelnden Körperteils entsprechen.

Hierdurch ergibt sich eine Vereinfachung bei der Herstellung, der Lagerhaltung und dem Vertrieb der Wärmebehandlungsvorrichtung als kommerziellem Produkt, da das Produkt dann nur noch in wenigen oder sogar nur noch in einer Version hergestellt, vorgehalten und ausgeliefert werden muss, ohne dass die Körpermaße der Patienten, insbesondere deren verschiedene Handgrößen, berücksichtigt werden müssen ("one size fits all").

Ein erfindungsgemäßes Wärmebehandlungssystem zur Verwendung bei der wärmetherapeutischen Behandlung eines Teiles des menschlichen Körpers weist eine erfindungsgemäße Wärmebehandlungsvorrichtung sowie eine Fluidversorgungseinrichtung auf, welche mit der ersten Zuleitung und der zweiten Zuleitung der Wärmebehandlungsvorrichtung fluidleitend verbindbar ist und welche dazu eingerichtet ist, ein wärmeübertragendes Fluid auf eine für die Wärmebehandlung geeignete Temperatur, insbesondere eine Temperatur zwischen +10 °C und +35 °C, zu temperieren, das derart temperierte wärmeübertragende Fluid dem Wärmeübertragungskörper der Wärmebehandlungsvorrichtung über die erste Zuleitung zuzuführen und das wärmeübertragende Fluid von dem Wärmeübertragungskörper über die zweite Zuleitung abzuführen.

Weitere vorteilhafte Ausgestaltungen der Erfindung werden in der nachfolgenden Beschreibung in Zusammenhang mit den Zeichnungen erläutert. Dabei zeigen:
- Fig. 1:: eine erfindungsgemäße Wärmebehandlungsvorrichtung in Handschuhform im aufgeklappten Zustand;
- Fig. 2:: die Wärmebehandlungsvorrichtung gemäß Fig. 1 im zusammengeklappten Zustand;
- Fig. 3:: einen faltenartigen Übergangsbereich des Wärmeübertragungskörpers in drei verschiedenen Ausführungen im Querschnitt.

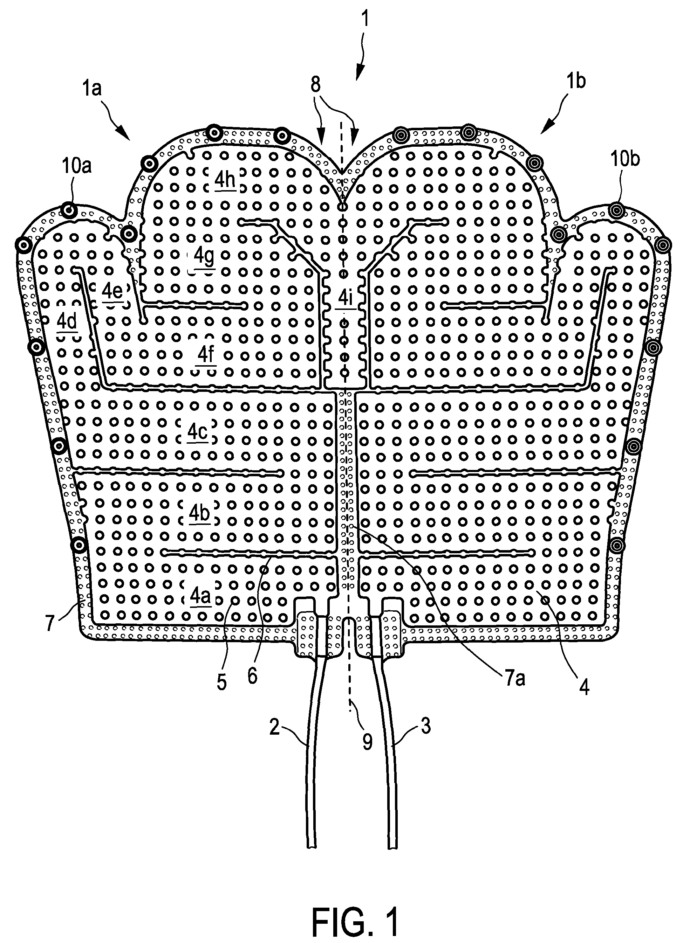

Fig. 1 zeigt eine erfindungsgemäße Wärmebehandlungsvorrichtung mit einem Wärmeübertragungskörper 1 im aufgeklappten Zustand, wodurch die beiden symmetrischen Hälften 1a, 1 b des Wärmeübertragungskörpers 1 sichtbar sind. Weiterhin weist die Wärmebehandlungsvorrichtung eine erste Zuleitung 2 und eine zweite Zuleitung 3 auf, durch welche ein wärmeleitendes, temperiertes Fluid, insbesondere erwärmtes oder gekühltes Wasser, durch eine (nicht gezeigte) Fluidversorgungseinrichtung zu- bzw. abführbar ist.

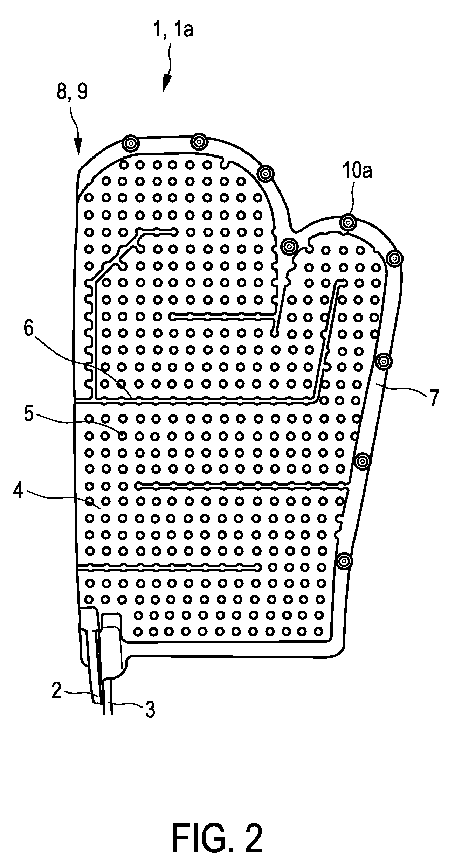

Der Wärmeübertragungskörper 1 lässt sich entlang einer Faltenlinie 9 zusammenklappen und nimmt im zusammengeklappten Zustand die Form eines Fausthandschuhs an, wie in Fig. 2 zu sehen.

Der Wärmeübertragungskörper 1 weist eine erste flexible Materiallage in Form einer Folie 11 und eine zweite flexible Materiallage in Form einer Folie 12 auf, welche deckungsgleich angeordnet sind und von denen in der Aufsicht in Fig. 1 lediglich die Innenseite der ersten Folie 11, d. h. die Hautkontaktfläche, sichtbar ist. Die beiden Folien 11, 12 sind vorzugsweise aus Kunststoff gefertigt. Im Ausführungsbeispiel gemäß Fig. 1 und 2 sind die beiden Folien 11, 12 weitgehend transparent.

An ihren äußeren Rändern sind die beiden Folien 11, 12 durch eine Randschweißnaht 7, welche nahezu lückenlos um den gesamten Wärmeübertragungskörper 1 herum verläuft, fluiddicht zusammengeschweißt. Außerdem verläuft eine Mittelschweißnaht 7a, welche etwa die gleiche Breite wie die Randschweißnaht 7 aufweist, auf der Faltenlinie 9 etwa bis zur Mitte der Faltenlinie 9 ins Innere des Wärmeübertragungskörpers 1 hinein.

Die Randschweißnaht 7 wird lediglich an den Durchführungsstellen einer ersten Zuleitung 2 und einer zweiten Zuleitung 3 unterbrochen, wo sie stark verbreitert ist, um die Enden der ersten und der zweiten Zuleitung 2, 3 sicher zu halten und fluiddicht abzudichten. Die Durchführungsstellen der ersten und der zweiten Zuleitung 2, 3 sind im Bereich des in Fig. 1 unteren Endes des Wärmeübertragungskörpers 1, entsprechend der am Handgelenk offenen Seite des Fausthandschuhs, angeordnet.

Auf der Randschweißnaht 7 sind in bestimmten, teilweise äquidistanten Abständen Druckknopfhälften 10a, 10b angebracht, und zwar auf der ersten Hälfte 1a des Wärmeübertragungskörpers 1 die steckerartigen und in der zweiten Hälfte 1 b die buchsenartigen Hälften. Nach dem Zusammenklappen des Wärmeübertragungskörpers 1 lässt sich jeweils eine steckerartige Druckknopfhälfte 10a in eine buchsenartige Druckknopfhälfte 10b einrasten. Dadurch nimmt der Wärmeübertragungskörper 1 die in Fig. 2 gezeigte Form eines Fausthandschuhs an, in welchen der Patient seine Hand stecken kann, um die Wärmetherapie anzuwenden.

Auch könnten die steckerartigen und/oder die buchsenartigen Druckknopfhälften 10a, 10b versetzt, insbesondere jeweils in mehreren Reihen, angeordnet sein, damit die Druckknopfhälften 10a, 10b auf verschiedene Weise ineinander einrasten können und sich dadurch für den Wärmeübertragungskörper 1 verschiedene Handschuhgrößen ergeben.

Die erste und die zweite Folie 11, 12 sind auf ihrer gesamten Fläche, abgesehen von der Randschweißnaht 7 und der Mittelschweißnaht 7a, durch eine Vielzahl von Schweißpunkten 5 miteinander verbunden, welche in einem quadratischen Raster angeordnet sind. Der Abstand zwischen benachbarten Schweißpunkten in (auf die Darstellung in Fig. 1 und 2 bezogen) horizontaler oder vertikaler Richtung wird im Folgenden als "Rasterabstand r" bezeichnet.

Durch weitere Schweißnähte im Flächeninneren der ersten und zweiten Folie 11, 12, durch welche die erste und die zweite Folie 11, 12 fluiddicht miteinander verbunden sind, ist eine Mehrzahl von Kanalwänden 6 gebildet. Die Kanalwände 6 bestehen zum größten Teil aus sich aneinander anschließenden, geradlinigen Abschnitten. Insbesondere verbinden die Kanalwände 6 jeweils eine Anzahl von benachbarten Schweißpunkten miteinander.

Durch die Kanalwände 6 wird in dem Wärmeübertragungskörper 1 zwischen der ersten und der zweiten Folie 11, 12 ein einziger, durchgehender, fluidleitender Kanal 4 gebildet.

Der Kanal 4 beginnt in der ersten Hälfte 1a des Wärmeübertragungskörpers 1 am Ende der ersten Zuleitung 2 und überdeckt zunächst die untere Hälfte der Hälfte 1a in Form von drei aufeinanderfolgenden, hin- und herlaufenden, geradlinigen und in Fig. 1 horizontal angeordneten Abschnitten 4a, 4b, 4c. Die Abschnitte 4a, 4b, 4c und auch deren Übergangsbereiche zueinander haben jeweils eine Breite von etwa 5 r, also dem Fünffachen das Rasterabstandes r, jeweils gemessen quer zur Strömungsrichtung innerhalb des Kanals 4. Die Abschnitte 4a, 4b, 4c sind in dem Fausthandschuh im Bereich der Handfläche angeordnet. Die Abschnitte 4a, 4b, 4c haben jeweils eine Länge von etwa 16 r bis 17 r.

Sodann geht der Kanal 4 in zwei aufeinanderfolgende, hin- und herlaufende, geradlinige und in Fig. 1 annähernd vertikal angeordnete Abschnitte 4d, 4e über, welche jeweils etwa eine Breite von etwa 3 r haben. Die Abschnitte 4d, 4e sind in dem Fausthandschuh im Bereich des Daumens angeordnet, wobei sie am äußeren Ende des Daumens abgerundet sind. Die Abschnitte 4d, 4e haben jeweils eine Länge von etwa 10 r.

Daran schließen sich wiederum zwei aufeinanderfolgende, hin- und herlaufende, geradlinige und in Fig. 1 horizontal angeordnete Abschnitte 4f, 4g mit einer Breite von jeweils etwa 5 *r* an, welche in dem Fausthandschuh im Bereich der inneren bzw. der mittleren Glieder der vier Finger (Zeigefinger bis kleiner Finger) angeordnet sind. Der Anfangsbereich des Abschnitts 4g ist etwa im Bereich des mittleren Glieds des kleinen Fingers abgeschrägt. Der Abschnitt 4f hat eine Länge von etwa 14 rund der Abschnitt 4g eine Länge von etwa 11 r, wobei die genannte Abschrägung nicht berücksichtigt ist.

Es folgt ein in Fig. 1 im Wesentlichen horizontal angeordneter und an seinem Ende auf der der Abschrägung des Abschnitts 4g gegenüberliegenden Seite schräg nach unten verlaufender Abschnitt 4h mit einer Breite bis zu etwa 5 r, welcher in dem Fausthandschuh im Bereich der äußeren Glieder der vier Finger angeordnet ist. Der obere Rand des Abschnitts 4h wird von einem Abschnitt der Randschweißnaht 7 gebildet und ist - den verschiedenen Längen der vier Finger entsprechend - abgerundet. Abschnitt 4h hat eine Länge von etwa 12 r, wobei die genannte Abrundung nicht berücksichtigt ist.

Hieran schließt sich ein in einem faltenartigen Übergangsbereich 8 des Wärmeübertragungskörpers 1 angeordneter Abschnitt 4i an, dessen Mittellinie quer zur Strömungsrichtung auf der Faltenlinie 9 liegt. Der Abschnitt 4i hat eine Breite von etwa 11 r, ist also gegenüber den vorhergehenden Abschnitten mehr als doppelt so breit. Auf diese Weise ist die Fluidmenge, welche pro Zeiteinheit in Abschnitt 4i über die Faltenlinie 9 fließen kann, etwa gleich groß wie in den vorhergehenden Abschnitten 4a bis 4h des Kanals 4, auch wenn sich in Abschnitt 4i der Abstand zwischen der ersten und der zweiten Folie 11, 12 durch das Zusammenklappen des Wärmeübertragungskörpers 1 entlang der Faltenlinie 9 verringert.

Der Abschnitt 4i hat eine Länge von nur etwa 2,5 r. Damit beträgt die Länge des Abschnitts 4i im Ausführungsbeispiel gemäß Fig. 1 nur etwa ein Viertel der Breite von etwa 11 rund ist somit deutlich kleiner als die Breite.

Der vorhergehende Abschnitt 4h mündet in Abschnitt 4i in einem Winkel von etwa 45 Grad. Der größte Teil der Breite von Abschnitt 4i - nämlich etwa 7 *r* von der Gesamtbreite von etwa 11 *r -* ist in Strömungsrichtung seitlich gegenüber dem Ausgang des Abschnitts 4h versetzt, und zwar in die zum Inneren des Wärmeübertragungskörpers 1 hin gerichtete Seite.

Der Verlauf des Kanals 4 in der zweiten Hälfte 1 b des Wärmeübertragungskörpers 1 ist symmetrisch zu dessen Verlauf in der ersten Hälfte 1a und wird daher hier nicht noch einmal beschrieben. Der Kanal 4 endet in der zweiten Hälfte 1 b am Beginn der zweiten Zuleitung 3.

Fig. 2 zeigt den Wärmeübertragungskörper 1 aus Fig. 1 im zusammengeklappten Zustand, wodurch nur die Hälfte 1 a und insbesondere die äußere Seite der zweiten Folie 12 mit den Rückseiten der steckerartigen Druckknopfhälften 10a sichtbar ist. Durch das Zusammenklappen hat der Wärmeübertragungskörper 1 in Fig. 2 die Form eines Fausthandschuhs angenommen.

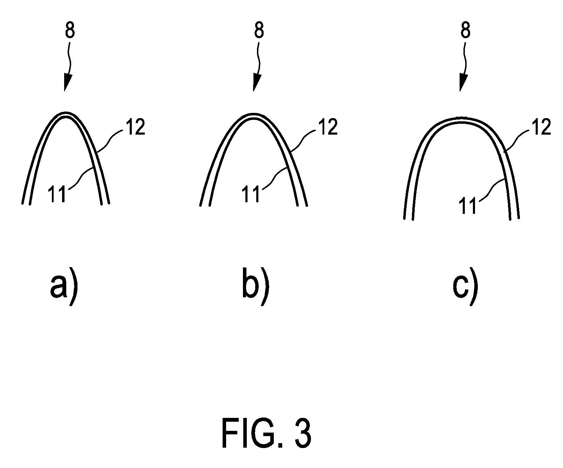

In Fig. 3 sind drei verschiedene Ausführungen einen faltenartigen Übergangsbereich 8 des Wärmeübertragungskörpers 1 im Querschnitt dargestellt, wobei der Biegeradius der ersten und der zweiten Folie 11, 12 von Fig. 3a) bis Fig. 3c) abnimmt.

In allen drei Ausführungen ist zu erkennen, dass der Abstand zwischen der ersten und der zweiten Folie 11, 12 in dem faltenartigen Übergangsbereich 8 geringer ist als außerhalb dieses Bereichs, d. h. an den unteren Enden des dargestellten Bereichs. Dies bewirkt die oben genannte Verringerung der Fluidmenge, die pro Zeiteinheit durch den Übergangsbereich 8 strömen kann.

### Bezugszeichenliste

- 1: Wärmeübertragungskörper
- 1a: Erste Hälfte des Wärmeübertragungskörpers
- 1b: Zweite Hälfte des Wärmeübertragungskörpers
- 2: Erste Zuleitung
- 3: Zweite Zuleitung
- 4: Kanal
- 4a - 4i: Kanalabschnitte
- 5: Schweißpunkt
- 6: Kanalwand
- 7: Randschweißnaht
- 7a: Mittelschweißnaht
- 8: Faltenartiger Übergangsbereich
- 9: Faltenlinie
- 10a: Steckerartige Druckknopfhälfte
- 10b: Buchsenartige Druckknopfhälfte
- 11: Erste Folie
- 12: Zweite Folie

## Patentansprüche

1. Wärmebehandlungsvorrichtung zur Verwendung bei der wärmetherapeutischen Behandlung eines Teiles des menschlichen Körpers, insbesondere zur Verwendung bei der Behandlung von Gewebeschädigungen nach operativen Eingriffen, von Verletzungen, von Entzündungen und von chronischen Erkrankungen des rheumatischen Formenkreises, mit einem Wärmeübertragungskörper (1), welcher aufweist:
- wenigstens eine erste flexible Materiallage (11) mit wenigstens einer ersten Hautkontaktfläche, welche zumindest teilweise dem zu behandelnden Körperteil zugewandt ist, und mit wenigstens einer von der ersten Hautkontaktfläche abgewandten ersten Fluidkontaktfläche,
- wenigstens eine zweite flexible Materiallage (12) mit wenigstens einer zweiten Fluidkontaktfläche, die zwischen sich und der ersten Fluidkontaktfläche der ersten flexiblen Materiallage (11) zumindest teilweise einen oder mehrere von einem wärmeübertragenden Fluid durchströmbare Strömungsräume (4) begrenzt,
wobei die Wärmebehandlungsvorrichtung weiterhin aufweist:
- wenigstens eine erste Zuleitung (2), um dem Wärmeübertragungskörper (1) ein wärmeübertragendes Fluid zuzuführen,
- wenigstens eine zweite Zuleitung (3), um ein wärmeübertragendes Fluid von dem Wärmeübertragungskörper (1) abzuführen,
wobei der Wärmeübertragungskörper (1) wenigstens eine erste Form aufweist oder eine solche erste Form annehmen kann, in welcher er den zu behandelnden Körperteil zumindest teilweise umschließt.

2. Wärmebehandlungsvorrichtung nach Anspruch 1, **dadurch gekennzeichnet, dass** der zu behandelnde Körperteil eine Hand ist und der Wärmeübertragungskörper (1) eine Handschuhform aufweist oder annehmen kann.

3. Wärmebehandlungsvorrichtung nach Anspruch 2, **dadurch gekennzeichnet, dass** der Wärmeübertragungskörper (1) eine der folgenden Formen aufweist oder annehmen kann:
- die Form einer die Hand umschließenden Hülle ohne Unterteilungen des Innenraums;
- die Form eines Handschuhs mit teilweise abgeteilten Umhüllungen für wenigstens einen Finger, insbesondere die Form eines Fausthandschuhs mit einer teilweise abgeteilten Umhüllung für den Daumen;
- die Form eines Fingerhandschuhs mit teilweise abgeteilten Umhüllungen für jeden Finger;
- die Form eines zumindest teilweise fingerlosen Handschuhs, bei dem wenigstens ein Finger nicht umschlossen ist.

4. Wärmebehandlungsvorrichtung nach wenigstens einem der vorhergehenden Ansprüche, **dadurch gekennzeichnet, dass** innerhalb wenigstens eines Strömungsraumes (4) wenigstens ein auf Zug und Druck beanspruchbares Verbindungselement (5) zwischen der ersten Fluidkontaktfläche und der zweiten Fluidkontaktfläche angeordnet ist.

5. Wärmebehandlungsvorrichtung nach Anspruch 4, **dadurch gekennzeichnet, dass** der Wärmeübertragungskörper (1) eine Vielzahl von Verbindungselementen (5) aufweist, welche zumindest teilweise in einem, vorzugsweise rechteckigen, weiter vorzugsweise quadratischen, Raster zwischen der ersten Fluidkontaktfläche und der zweiten Fluidkontaktfläche angeordnet sind.

6. Wärmebehandlungsvorrichtung nach wenigstens einem der vorhergehenden Ansprüche, **dadurch gekennzeichnet, dass** zwischen der ersten Fluidkontaktfläche und der zweiten Fluidkontaktfläche verlaufende Begrenzungen der Strömungsräume (4) zumindest teilweise durch linienförmige Kontaktierungen (6) der ersten Fluidkontaktfläche mit der zweiten Fluidkontaktfläche gebildet sind.

7. Wärmebehandlungsvorrichtung nach wenigstens einem der Ansprüche 4 und 5 und nach Anspruch 6, **dadurch gekennzeichnet, dass** zumindest zwei benachbarte Verbindungselemente (5), insbesondere die Mittelpunkte der beiden Verbindungselemente (5), durch eine der linienförmigen Kontaktierungen (6), insbesondere geradlinig, verbunden sind.

8. Wärmebehandlungsvorrichtung nach wenigstens einem der Ansprüche 6 und 7, **dadurch gekennzeichnet, dass** wenigstens eine der linienförmigen Kontaktierungen (6) wenigstens einen geradlinigen Abschnitt aufweist, vorzugsweise dass die linienförmigen Kontaktierungen (6) zu mehr als 50 % ihrer Gesamtlänge, weiter vorzugsweise zu mehr als 90 % ihrer Gesamtlänge aus geradlinigen Abschnitten bestehen.

9. Wärmebehandlungsvorrichtung nach wenigstens einem der vorhergehenden Ansprüche, **dadurch gekennzeichnet, dass** der Wärmeübertragungskörper (1) zusammenklappbar ist, insbesondere dass er zwei im Wesentlichen zueinander symmetrisch geformte Hälften (1a, 1 b) aufweist, welche im zusammengeklappten Zustand des Wärmeübertragungskörpers (1) im Wesentlichen miteinander in Deckung gebracht sind.

10. Wärmebehandlungsvorrichtung nach Anspruch 9, **dadurch gekennzeichnet, dass** der Wärmeübertragungskörper (1) einen faltenartigen Übergangsbereich (8) mit einer Faltenlinie (9) aufweist, wobei der Wärmeübertragungskörper (1) entlang der Faltenlinie (9) zusammenklappbar ist und sich wenigstens ein Strömungsraum (4) über die Faltenlinie (9) hinweg erstreckt.

11. Wärmebehandlungsvorrichtung nach Anspruch 10, **dadurch gekennzeichnet, dass** sich mehrere Strömungsräume oder mehrere Abschnitte desselben Strömungsraumes über die Faltenlinie (9) hinweg erstrecken.

12. Wärmebehandlungsvorrichtung nach Anspruch 11, **dadurch gekennzeichnet, dass** mehrere sich über die Faltenlinie (9) hinweg erstreckende Abschnitte desselben Strömungsraumes auf einer oder auf beiden Seiten der Faltenlinie (9) durch je einen Abschnitt dieses Strömungsraumes verbunden sind, welcher jeweils im Wesentlichen entlang der Faltenlinie (9) verläuft.

13. Wärmebehandlungsvorrichtung nach Anspruch 10, **dadurch gekennzeichnet, dass** ein sich über die Faltenlinie (9) hinweg erstreckender Strömungsraum gleichzeitig auf beiden Seiten der Faltenlinie (9) im Wesentlichen entlang der Faltenlinie (9) verläuft.

14. Wärmebehandlungsvorrichtung nach einem der Ansprüche 10 bis 13, **dadurch gekennzeichnet, dass** wenigstens ein Verbindungselement (5) auf der Faltenlinie (9) innerhalb eines Strömungsraumes (4), welcher sich über die Faltenlinie (9) hinweg erstreckt, angeordnet ist.

15. Wärmebehandlungsvorrichtung nach einem der Ansprüche 10 bis 14, **dadurch gekennzeichnet, dass** die entlang der Faltenlinie (9) gemessene Gesamtbreite eines sich über die Faltenlinie (9) hinweg erstreckenden Strömungsraumes (4) größer als, insbesondere wenigstens doppelt so groß wie, die senkrecht zur Strömungsrichtung gemessene Gesamtbreite dieses Strömungsraumes (4) in einem in Strömungsrichtung gesehen unmittelbar vor oder nach dem faltenartigen Übergangsbereich(8) angeordneten Abschnitt dieses Strömungsraumes (4) ist.

16. Wärmebehandlungsvorrichtung nach wenigstens einem der vorhergehenden Ansprüche, **dadurch gekennzeichnet, dass** wenigstens zwei Abschnitte (1 a, 1 b), insbesondere zwei im Wesentlichen zueinander symmetrisch geformte Hälften, des Wärmeübertragungskörpers (1) durch wenigstens ein Fixierungselement (10a, 10b), insbesondere durch einen Druckknopf, einen Klettverschluss oder eine Schlaufe zum Binden, miteinander lösbar verbindbar sind.

17. Wärmebehandlungsvorrichtung nach Anspruch 16, **dadurch gekennzeichnet, dass** die wenigstens zwei Abschnitte (1 a, 1 b) des Wärmeübertragungskörpers (1) auf verschiedene Weise miteinander lösbar verbindbar sind, so dass dabei verschiedene Größen des Wärmeübertragungskörpers (1) entstehen, welche insbesondere verschiedenen Größen des zu behandelnden Körperteils entsprechen.

18. Wärmebehandlungssystem zur Verwendung bei der wärmetherapeutischen Behandlung eines Teiles des menschlichen Körpers mit einer Wärmebehandlungsvorrichtung nach wenigstens einem der vorhergehenden Ansprüche und einer Fluidversorgungseinrichtung, welche mit der wenigstens einen ersten Zuleitung (2) und der wenigstens zweiten Zuleitung (3) der Wärmebehandlungsvorrichtung fluidleitend verbindbar ist und welche dazu eingerichtet ist, ein wärmeübertragendes Fluid auf eine für die Wärmebehandlung geeignete Temperatur, bevorzugt eine Temperatur zwischen +10 °C und +35 °C, weiter bevorzugt zwischen +10° und +20° und besonders bevorzugt zwischen +14° und +16° und ganz besonders bevorzugt um ca. 15° zu temperieren, das derart temperierte wärmeübertragende Fluid dem Wärmeübertragungskörper (1) der Wärmebehandlungsvorrichtung über die erste Zuleitung (2) zuzuführen und das wärmeübertragende Fluid von dem Wärmeübertragungskörper (1) über die zweite Zuleitung (3) abzuführen.
